# EUROPÄISCHE PATENTSCHRIFT

(11) **EP 0 864 328 B1**
(45) Veröffentlichungstag und Bekanntmachung des Hinweises auf die Patenterteilung: **19.02.2003**
(21) Anmeldenummer: 97113756.7
(22) Anmeldetag: 08.08.1997
(51) Int. Cl.: A61K 33/00, A61K 47/02, A61K 9/00, A61K 9/107, A61M 5/142

(54) **Vorrichtung zur gesteuerten Anästhesie, Analgesie und/oder Sedierung**
Apparatus for controlled anaesthesia, analgesia and/or sedation
Appareil pour l'anesthésie, l'analgésie et/ou la sédation contrôlée

(30) Priorität: 10.03.1997 DE 19709704
(43) Veröffentlichungstag der Anmeldung: 16.09.1998
(73) Patentinhaber: Georgieff, Michael, Dr., 89081 Ulm/Ermingen (DE)
(72) Erfinder: Georgieff, Michael, Dr., 89081 Ulm/Ermingen (DE)
(74) Vertreter: Wibbelmann, Jobst, Dr., Dipl.-Chem.

(56) Entgegenhaltungen:
- EP-A- 0 523 315
- WO-A-85/00011
- WO-A-95/27438
- DE-A- 1 667 926
- DE-A- 3 940 389
- DE-A- 4 100 782
- DE-A- 4 411 533

## Beschreibung

Die Erfindung betrifft eine Vorrichtung, mit der sich die Anästhesie, Analgesie und/oder Sedierung eines Patienten steuern lässt.

Unter Steuerung wird hier verstanden, dass der Zustand eines Patienten (anästhesiert, analgesiert und/oder sediert), ausgehend von einem Ist-Zustand des Patienten in Richtung auf einen erforderlichen oder anzustrebenden Zustand, in kürzest möglichen Zeiträumen verändert werden kann. Das heißt z.B., dass im Falle einer Narkose die Zustände (1) Analgesie, (2) Bewusstseinsverlust und (3) Muskelerschlaffung in kürzester Zeit erreicht werden und dass der Übergang vom narkotisierten Zustand in das volle Bewusstsein schnell und komplikationslos abläuft. Steuerung bedeutet weiterhin, dass ein einmal erreichter Zustand über lange Zeiträume (Stunden bis Tage) stabil gehalten wird. Das heißt auch bei sich drastisch verändernden Bedingungen bleibt der Zustand erhalten und es kann problemlos nachgesteuert werden.

Wenn eine solche Steuerung zuverlässig und komplikationsfrei verlaufen soll, sind zunächst einmal bestimmte Anforderungen an einen Wirkstoff zu stellen. Der Wirkstoff muss sich beispiels-weise durch einen schnellen Wirkungseintritt (wenige Sekunden) auszeichnen. Andererseits muss die Wirkung aber auch schnell abklingen (beispielsweise 1 - 3 Minuten; Reversibilität; sämtliche Ausfallerscheinungen müssen nach Beendigung der Narkose wieder verschwinden). Weiterhin ist eine ausreichende (beispielsweise anästhesiologische) Sicherheitbreite zu fordern. Die zur Erreichung des gewünschten Zustands (beispielsweise Verlust der Schmerzempfindung und Bewußtseins-verlust) erforderliche Konzentration sollte um ein Vielfaches niedriger sein als diejenige, durch die vitale Funktionen des Patienten beeinträchtigt werden. Schließlich kommt es aber auch auf die Steuerbarkeit an, d.h. durch Variation der Konzentra-tion oder der Infusionsrate lässt sich den Zustand vertiefen, abflachen oder beenden. Bei länger andauernden Operationen (z.B. Operationen, die mehr als 10 Sekunden in Anspruch nehmen) ist zusätzlich zu fordern, dass der Wirkstoff in höheren Konzentrationen über einen längeren Zeitraum gegeben werden kann, ohne dass es zu beträchtlichen Nebenwirkungen kommt.

Die sich zur Zeit im Einsatz befindlichen intravenösen applizierbaren Narkosemittel zeichnen sich zwar durch einen sofortigen Wirkungseintritt aus, zeigen aber regelmäßig ein Bündel von Nachteilen. Hervorzuheben ist, dass insbesondere Propofol und Etomidat keine analgetische Wirkung haben und schlecht steuerbar sind. Weitere Nachteile dieser Injektionsnarkosemittel liegen in schlecht einzuschätzenden Nebenwirkungen (beispielsweise Blutdruckabfall, Bradykardie, Rigidität, allergische Reaktionen) und zum Teil gravierenden Kontraindi-kationen. Schließlich kommt es auch bei der totalintravenösen Anästhesie (TIVA) mit Propofol insbesondere bei längerer Narkose häufig zu protahiertem Erwachen und Desorientiertheit. Es zeigt sich also, dass die zur Zeit bekannten intravenös verabreichbaren Wirkstoffe den Anforderungen nicht genügen.

Wirkstoffkombinationen nach dem Stand der Technik stellen keine Lösung dieses Problems dar. Insbesondere bei Anästhetika ist bekannt, dass Kombinationen zu pharmakokinetischen und pharmakodynamischen Wechselwirkungen führen, die ganz sicher unzureichend bei der Aufrechterhaltung der Anästhesie beherrscht werden können. In Folge einer unterschiedlichen Pharmakokinetik und Pharmakodynamik der jeweils eingesetzten Wirksubstanzen zu einem bestimmten Moment im Verlauf der Anästhesie ist eine richtige Einstellung der Konzentration und/oder Infusionsrate nicht möglich. Mit anderen Worten, bei Wirkstoffkombinationen in einem intravenösen Präparat entspricht die Gesamtwirkung praktisch nie der Summe der Einzelwirkungen. Deshalb erfüllen solche Kombinationspräparate nicht die Anforderung der Steuerbarkeit.

Es besteht somit ein Bedürfnis nach einer als Einzelsubstanz oder in Kombination mit anderen Wirkstoffen einzusetzenden Substanz, die die oben formulierten Anforderungen erfüllt.

Eine ganz exakte Steuerung der Anästhesie, insbesondere die Aufrechterhaltung der Anästhesie, erfordert, dass zu jedem Zeitpunkt eine bestimmte Konzentration des Wirkstoffs im Blut eines Patienten eindeutig messbar ist. Bei überschaubaren einfachen Operationsverläufen und bekannter Pharmakokinetik ist eine begrenzte Steuerung mittels mehrstufiger Infusionsregime beispielsweise mit Propofol möglich. Solche Regime sind aber inflexibel und insbesondere dann nicht geeignet, wenn der Wirkstoff in gesteuerter Weise bei sich ändernden anästheti-schen und operativen Vorgaben verabreicht werden muss.

Wegen der fehlenden Flexibilität bei manuellen Infusionsregimen und der recht komplexen mathematischen Modelle für die Pharmakokinetik der bekannten Wirkstoffe hat man computergesteuerte Infusionssysteme entwickelt. Diese Computersysteme werden mit einer mathematischen Lösung für das pharmakokinetische Modell eines Patienten bezüglich des eingesetzten Wirkstoffes, beispielsweise Propofol, programmiert. Der Computer errechnet dann die Infusionsrate, die zum Erreichen und Aufrechterhalten einer theoretischen Zielblutkonzentration erforderlich ist. Dieser Zielwert wird z.B. von einem Anästhesisten bestimmt und eingestellt. Der Computer steuert dann auch die Infusionsrate, mit der der Wirkstoff an einen Patienten verabreicht wird. Diese Art der Steuerung wird auch als Target-Controlled Infusion (TCI) bezeichnet.

Es gibt aber immer eine Ungewissheit hinsichtlich der Konzentration des Wirkstoffs, da sich die Pharmakokinetik von Patient zu Patient sich ändert. Tatsächlich wurde beobachtet, dass in der Praxis recht unterschiedliche Zielkonzentrationen von Anästhesisten bestimmt wurden. Daraus ergibt sich, dass ein erhebliches Bedürfnis nach einem System besteht, das einen bestimmten Zustand während einer Anästhesie in Abhängigkeit von den tatsächlichen Erfordernissen bei einem Patienten während einer Operation einstellen bzw. ansteuern kann. Die erheblichen Unterschiede bezüglich der Zielkonzentration des Wirkstoffs im Blut und die beträchtliche Varianz, die im Verlauf von Operationen mit verschiedenen Patienten und den zusätzlich zur Anwendung kommenden Pharmaka beobachtet wird, lassen den Rückschluss zu, dass die TCI den Anforderungen an eine effektive Steuerung noch nicht in jeder Hinsicht genügt.

In der Entwicklung befinden sich zur Zeit Systeme, mit denen man den Grad einer Anästhesie genauer einstellen kann. Hierbei handelt es sich um geschlossene Kreislaufsysteme, bei denen die Verabreichung des Injektionsanästhetikums in Abhängigkeit von der tatsächlich ermittelten Tiefe einer Anästhesie gesteuert wird (sogenannte Closed Loop Anesthesia Systems (CLAN)). Bei diesen Systemen ist aber ein beträchtlicher apparativer Aufwand erforderlich, um bei einem Patienten die Wirkung des Anästhe-tikums, d.h. die Tiefe der Anästhesie, exakt zu bestimmen.

Zusammengefasst, es besteht somit nicht nur ein Bedürfnis nach einem anästhetisch, analgetisch und sedierend wirkenden Stoff, der allen Anforderungen genügt, um in einem reellen Steuerungssystem (TCI oder CLAN) wie zuvor diskutiert eingesetzt werden zu können. Darüberhinaus besteht ein Bedürfnis nach einfacheren Systemen, die auch ohne komplexe Rechnerprogramme und/oder aufwendige Messgeräte (nebst Auswertungsprogrammen) auskommen können, die im Gegensatz zu den bekannten Systemen den reellen Zustand ansprechen (beispielsweise reelle Konzentration im Blut).

Die Aufgabe der Erfindung besteht darin, eine Vorrichtung (bzw. Einrichtung) bereitzustellen, durch die eine gesteuerte Anästhesie, Analgesie und/oder Sedierung gewährleistet werden kann.

Diese Aufgabe wird gelöst durch eine Vorrichtung, die dadurch gekennzeichnet ist, dass sie einen Behälter mit einer Emulsion, die Xenon in einer anästhesierend, analgesierend oder sedierend wirksamen Menge enthält, und Mittel zur gesteuerten Abgabe der Emulsion an den Patienten aufweist. Diese Vorrichtung dient dazu, zeitlich gesteuert eine xenonhaltige Präparation intravenös oder arteriell an einen Patienten abzugeben. Zeitlich gesteuert bedeutet hier, dass über einen vorgegebenen Zeitraum, beispielsweise 2 Minuten oder sogar 1 bis 2 Stunden und darüber (bis Tage), der beispielsweise in einem Operationsverlauf erforderliche Zustand (anästhesiert, analgesiert und/oder sediert) jeweils exakt angesteuert werden kann. Dies wird beispielsweise erzielt durch das Anpeilen einer bestimmten endexpiratorischen Xenon-Konzentration, die derjenigen des Blutes entspricht. Im allereinfachsten Fall ist der Behälter mit der Emulsion eine Spritze. Das Mittel zur gesteuerten Abgabe ist dann der Spritzenkolben, der beispielsweise unter Zuhilfenahme eines sogenannten Perfusors mit einem solchen Druck beaufschlagt wird, der eine gesteuerte Abgabe der Emulsion ermöglicht (z.B. kontinuierliche intravenöse Verabreichung eines Volumens von 20 ml über 30 sec). Mit einer solchen Vorrichtung lässt sich die Aufrechterhaltung einer Anästhesie über kürzere Zeiträume (10 sec bis etwa 60 min) gewährleisten. Die Narkosetiefe, Analgesierung, Sedierung und/oder Muskelrelaxierung wird beispielsweise über die endexpiratorische Xenon-Konzentration genau eingestellt. Da die Pharmakokinetik des Wirkstoffes sehr viel einfacher ist als im Falle des Propofols sind keine abgestuften Infusionsregime erforderlich.

Eine weitere Ausführungsform umfasst einen Infusionsbeutel, der mit der flüssigen Präparation befüllt ist, einen Schlauch, der die Verbindung zum Patienten herstellt, und einen einfachen Regler zur Steuerung der Abgabe. Komplexere Ausgestaltungen umfassen elektronische Steuerungseinrichtungen und Pumpen, wie beispielsweise Infusionspumpen.

Die erforderlichen Einstellungen für die Infusion der ein Edelgas enthaltenden flüssigen Präparation lassen sich u.a. dadurch ermitteln, dass man beispielsweise den Ablauf einer Operation vorab bei einem Patienten simuliert. Das heißt bei einem Patienten wird zuvor die für einen bestimmten Zustand (anästhesiert/analgesiert/sediert) zugehörige Infusionsrate/ Konzentration bestimmt. Eine solche Bestimmung kann problemlos unmittelbar vor der eigentlichen Operation durchgeführt werden.

Die Erfindung geht zum Teil auf die überraschende Entdeckung zurück, dass mit einer xenonhaltigen (Kr, Ar, Xe) flüssigen Präparation ein anästhesierter, analgetischer oder sedierter Zustand leicht angesteuert werden kann.

Bei Xenon handelt es sich um ein einatomiges farb-, geruchund geschmackloses Edelgas der Ordnungszahl 54. Xenon ist fünfmal schwerer als Luft. Natürliches Xenon enthält des weiteren Isotope, beispielsweise die Isotope 124, 126, 128, 129, 130,
131, 132, 134 und 136. Daneben sind auch künstliche Isotope bekannt sowie Xenon 114, Xenon 133 und Xenon 142. Diese Isotope zerfallen mit Halbwertszeiten, die zwischen 1,2 Sekunden und etwa 40 Tagen liegen. Radioaktive Xenon-Isotope werden von der vorliegenden Erfindung nicht angesprochen.

Emulsionen im Sinne der vorliegenden Erfindung können aufgrund einer gewissen Lipophilie ein fettlösliches Gas, wie das erwähnte Xenon, leicht aufnehmen.

Um eine subanästhesierende Wirkung zu erreichen, genügt bereits eine Xenonbeladung der medizinischen Präparation von etwa 0,2 bis 0,3 ml Xenon pro ml Emulsion. Das bedeutet, dass eine analgesierende und/oder sedierende Wirkung bei Präparationen mit einen Xenongehalt von mindestens 0,2 ml/ml Emulsion gewährleistet ist. Eine antiinflammatorische Wirkung wird schon bei 0,1 ml/ml Emulsion beobachtet. Es hat sich gezeigt, dass
20 ml einer Emulsion, die 0,3 ml Xe pro ml Emulsion enthält, bei kontinuierlicher Infusion über 30 sec einen subanästhesier-ten Zustand bei einem etwa 85 kg schweren Patienten ermöglicht. Wenn man mit einer hochbeladenen Perfluorcarbonemulsion arbeitet, die beispielsweise 2 bis 4 ml Xenon pro ml Emulsion enthält, so wird zur Narkoseeinleitung beispielsweise 20 ml dieser Emulsion über 30 sec infundiert. Zur Aufrechterhaltung der Anästhesie reicht eine Infusionsrate von mindestens 7,5 ml/min. Insgesamt würden somit 470 ml Emulsion für eine 1-stündige Operation anfallen. Das entspricht einem Xenonvolumen bei einem Xenongehalt von 3 ml Xenon pro ml Emulsion von 1410 ml, also einem Bruchteil des Xenonverbrauchs bei einer Inhalationsnarkose (bezogen auf ein Körpergewicht von 85 kg wäre das ein Verbrauch von 16,6 ml pro kg in einer Stunde).

Darüber hinaus ist es möglich und unter Umständen auch vorteilhaft, in der Präparation neben dem Edelgas ein weiteres pharmakologisch wirksames Mittel vorzusehen. Dies kann beispielsweise ein intravenöses Sedativum oder Anästhetikum sein. Je nachdem ob es sich hierbei um ein wasserlösliches oder fettlösliches Mittel handelt, ist dieses dann in der wäßrigen Phase oder der Lipidphase neben dem Xenon vorhanden. Als besonders geeignet hierfür bietet sich 2,6-Diisopropylphenol an, wobei es sich um ein wirksames Anästhetikum handelt (beispielsweise 1,5 - 20 mg/ml). Geeignet ist auch Etomidat in Konzentrationen von 0,1 - 2 mg/ml (Hypnomidate®, ein Imidazol-5-carbonsäurederivat). Durch den Einsatz von gelöstem Xenon neben dem weiteren Anästhetikum lässt sich die zur Narkotisie-rung notwendige Konzentration von bsp. Diisopropylphenol oder Etomidat auf kleinere Werte absenken. So kann beispielsweise 1 ml erfindungsgemäße Fettemulsion (die etwa 0,1 g Fett pro ml Emulsion enthält) 2,5 - 20 mg 2,6-Diisopropylphenol enthalten, d. h. beispielsweise 2,5, 5,0, 7,5, 10, 15 oder 20 mg neben dem Xenon.

Es können ganz allgemein neben dem Xenon als anästhesierend, analgetisch oder sedierend wirkender Substanz weitere Anästhetika, Analgetika, Muskelrelaxanzien oder Sedativa vorhanden sein. Als weitere Anästhetika kommen z.B. Barbiturate (u.a. Barbital, Phenobarbital, Pentobarbital, Secobarbital, Hexobarbital und Thiopental) im allgemeinen und Opioide in Betracht. Bekannte Analgetika sind u.a. Verbindungen vom Typ des Morphins, z.B. Hydromorphon, Oximorphon, Codein, Hydrocodon, Thebacon, Thebain, Heroin. Weiterhin können synthetische Derivate des Morphins verwendet werden, z.B. Pethidin, Levomethadon, Dextromoramid, Pentazocin, Fentanyl, Alfentanil. Auch schwächer wirkende Analgetika können zum Einsatz kommen wie Anthranilsäurederivate (Flufenaminsäure, Mefenaminsäure), Acrylsäurederivate (Diclofenac, Tolmetin, Zomepirac), Arylpropylsäurederivate (Ibuprofen, Naproxen, Phenoprofen, Ketoprofen) und Indol- bzw. Indenessigsäurederi-vate (Indometacin, Sulindac). Als Muskelrelaxanzien können zentrale Muskelrelaxanzien eingesetzt werden wie beispielsweise Baclofen, Carisoprodol, Chlordiazepoxid, Chlormezanon, Chloroxazon, Dantrolen, Diazepam, Phenyramidol, Meprobamat, Phenprobamat, Orfenadrin. Sedativa, die erfindungsgemäß eingesetzt werden können, sind u.a. Benzodiazepinderivate wie Triazolam, Lormetazeban, Clotiazepam, Flurazepam, Nitrazepam, Flunitrazepam.

Flüssigkeiten, die lipophile Edelgase aufnehmen können, sind z.B. Blutersatzmittel, u.a. Perfluorcarbonemulsionen (z.B. Perflubron).

Es ist allgemein bekannt, dass Perfluorcarbonverbindungen ein hohe Löslichkeit hinsichtlich einer Vielzahl von Gasen haben. Eine Perfluorcarbonemulsion besteht beispielsweise aus bis zu 90 % (Gewicht/Volumen) Perflubron (C₈F₁₇). Zusätzlich sind Emulgatoren, beispielsweise Phospholipide aus Hühnereigelb erforderlich. Über diese Emulsionen, die sich erfindungsgemäß mit Xenon beladen lassen, wird beispielsweise bei J.A. Wahr et al. in Anesth. Analg. 1996, 82, 103-7, berichtet.

Geeignete Fluorocarbonemulsionen umfassen vorzugsweise 20 w/v % bis 125 w/v % einer hochfluorierten Kohlenwasserstoffverbindung, beispielsweise mehrfach fluorierte Bisalkylethene, cyclische Fluorkohlenstoffverbindungen wie Fluor-dekalin oder Perfluor-dekalin, fluoriertes Adamantan, perfluorierte Amine wie fluoriertes Tripropylamin und fluoriertes Tributylamin. Es können auch monobromierte Perfluorcarbone eingesetzt werden wie beispielsweise 1-Bromheptadecafluoroctan (C₈F₁₇Br), 1-Brompenta-decafluorheptan (C₇F₁₅Br) und 1-Bromtridecafluorhexan (C₆F₁₃Br). Darüber hinaus sind weitere Verbindungen brauchbar, wie unter anderem perfluoralkylierte Ether oder Polyether, z.B. (CF₃)₂ CFO(CF₂CF₂)₂ OCF(CF₃)₂, (CF₃)₂ CFO(CF₂CF₂)₃ OCF₂(CF₃), (CF₃)₂ CFO(CF₂CF₂)₂F, (CF₃)₂ CFO(CF₂CF₂)₃F und (C₆F₁₃)₂O.

Weiterhin sind chlorierte Derivate der zuvor erwähnten Perfluorcarbone einsetzbar.

Die Trägerkapazität der zuvor erwähnten Perfluorcarbonpräparation ist beträchtlich. Es wurden z.B. Xenonbeladungen von 1 bis 10 ml/ml mit einfachsten Mitteln erreicht. Beispiels-weise können diese Präparationen mit Edelgas durch einfaches Durchleiten des Gases beladen werden.

Weiterhin können Fettemulsionen eingesetzt werden, die in der Lipidphase gelöst oder dispergiert das lipophile Edelgas enthalten.

Es hat sich gezeigt, dass man Xenon in einer beträchtlichen Menge in einer Fettemulsion anreichern kann. So kann bereits mit einfachsten Mitteln Xenon in Konzentrationen von 0,2 bis 10 ml (Konzentrationen beziehen sich auf Standardbedingungen, d.h. 20°C und Normaldruck) und darüber pro ml Fettemulsion gelöst bzw. dispergiert werden. Die Xenon-Konzentration hängt von einer Vielzahl von Faktoren ab, insbesondere der Konzentration des Fetts. In der Regel wird man die Präparationen bis an die Sättigungsgrenze mit Xenon "beladen". Es können aber auch sehr geringe Konzentrationen vorliegen, soweit beispielsweise bei intravenöser Applikation eine pharmakologische Wirksamkeit noch beobachtet werden kann. Bei einer 10 %igen Fettemulsion können ohne weiteres Xenon-Konzentrationen von 0,3 bis 2 ml Xenon pro ml Fettemulsion erreicht werden. Selbstverständlich sind auch höhere Werte, wie z.B. 3, 4, 5, 6 oder 7 ml Xenon pro ml Fettemulsion erreichbar. Diese Fettemulsionen sind zumindest in gasdicht verschlossenen Behältern hinreichend stabil, so dass das Xenon sich während üblicher Lagerungszeiträume nicht wieder als Gas freisetzt.

Die Lipidphase der Präparation, die das Gas aufnimmt, d.h. lösen und/oder dispergieren kann, wird im wesentlichen durch sog. Fette gebildet, wobei es sich im wesentlichen um Ester von langkettigen und mittellangkettigen Fettsäuren handeln kann. Solche Fettsäuren, gesättigt oder ungesättigt, enthalten 8 bis 20 Kohlenstoffatome. Daneben können aber auch omega-3-oder omega-6-Fettsäuren eingesetzt werden, die bis zu 30 Kohlen-stoffatome enthalten können. Als veresterte Fettsäuren bieten sich insbesondere pflanzliche Öle an, wie z. B. Baumwoll-samenöl, Sojabohnenöl, Distelöl, Fischöl und dgl. Hauptbestand-teil dieser natürlich vorkommenden Öle sind die Triglyceride der Fettsäuren. Von besonderer Bedeutung sind Präparationen, die als sog. Öl-in-Wasser-Emulsionen vorliegen. Dabei macht der Fettanteil der Emulsion üblicherweise 5 bis 30 Gew.-%, vorzugs-weise 10 bis 20 Gew.-% aus. Neben dem Fett ist aber in der Regel ein Emulgator vorhanden, wobei sich Sojaphosphatide, Gelatine oder auch Eiphosphatid bewährt haben. Solche Emulsionen können hergestellt werden, indem das mit Wasser nicht mischbare Öl in Gegenwart des Emulgators, in der Regel ein oberflächenaktives Mittel, in Wasser emulgiert wird. Neben dem Wasser können auch andere polare Lösemittel, wie beispiels-weise Ethanol, Glycerin (Propylenglykol, Hexylenglykol, Polyethylenglykol, Glykolmonoether, ein mit Wasser mischbarer Ester, etc.) vorhanden sein. Das Edelgas kann bereits in einer vorausgehenden Verfahrensstufe in die Lipidphase eingebracht worden sein. Im einfachsten Fall bietet sich aber an, die fertiggestellte Emulsion mit dem Xenon zu beladen. Dies kann bei unterschiedlichen Temperaturen erfolgen, beispielsweise bei Temperaturen von 1°C bis zu Raumtemperatur. Hierbei ist es zuweilen hilfreich, das Gefäß, in dem sich die Emulsion befindet, mit einem Druck von beispielsweise bis zu 8 Atmosphären oder darüber zu beaufschlagen.

Erfindungsgemäß lassen sich Fettemulsionen einsetzen, wie sie bei der intravenösen Ernährung zum Einsatz kommen. Diese Fettemulsionen bestehen im wesentlichen aus einer geeigneten Fettgrundlage (Sojabohnenöl oder Sonnenblumenkernöl) und einem gutverträglichen Emulgator (Phosphatide). Allgemein gebräuchliche Fettemulsionen sind Intralipid®, Intrafat®, Lipofundin®S und Liposyn®. Genauere Angaben zu diesen Fettemulsionen kann man G. Kleinberger und H. Pamperl, Infusionstherapie, 108-117 (1983) 3, entnehmen. Die Fettemulsionen enthalten im allgemeinen noch Zusätze, die die Osmolarität der wäßrigen Phase, die die in Form von Liposomen vorliegende Fettphase umgibt, Blut-isoton machen. Hierzu kann man Glycerin und/oder Xylit verwenden. Darüber hinaus ist es häufig sinnvoll, der Fettemulsion ein Antioxidationsmittel zuzugeben, um eine Oxidation der ungesättigten Fettsäuren zu verhindern. Hierfür eignet sich insbesondere Vitamin E (DL-Tocopherol).

Als Lipidphase besonders vorteilhaft, insbesondere bei einer Öl-in-Wasser-Emulsion, sind sog. Liposomen, die sich aus den oben erwähnten Triglyceriden aber auch allgemein aus sog. Phospholipidmolekülen bilden lassen. Diese Phospholipidmoleküle bestehen im allgemeinen aus einem wasserlöslichen Teil, der durch mindestens eine Phosphatgruppe gebildet wird, und einem Lipidteil, der sich von einer Fettsäure bzw. deren Ester ableitet.

In der US-A-5 334 381 wird im Detail erläutert, wie man Liposomen mit Gas beladen kann. Ganz allgemein gesprochen wird eine Vorrichtung mit den Liposomen gefüllt, d.h. mit einer Öl-in-Wasser-Emulsion, und dann wird die Vorrichtung mit dem Gas darin unter Druck gesetzt. Dabei kann die Temperatur bis auf 1°C abgesenkt werden. Unter Druck löst sich das Gas allmählich auf und gelangt in die Liposomen. Bei einer Entspannung des Drucks kann es dann zur Ausbildung von kleinen Gasblasen kommen, die aber jetzt von den Liposomen eingekapselt werden. Somit ist es praktisch möglich, beispielsweise Xenongas oder andere Gase unter hyperbaren Bedingungen in einer Fettemulsion zu halten. Auch solche Präparationen können erfindungsgemäß verwendet werden, solange es nicht zur Ausbildung einer separaten Gasphase außerhalb der Liposomen kommt und vorausgesetzt, dass die angestrebte pharmakologische Wirkung eintritt.

Die Lipide, die die Liposomen ausbilden, können von natürlicher oder synthetischer Herkunft sein. Solche Materialien sind beispielsweise Cholesterol, Phosphatidylcholin, Phosphatidyl-ethanolamin, Phosphatidylserin, Phosphatidylglycerin, Phosphatidylinositol, Sphingomyelin, Glycosphingolipide, Glucolipide, Glycolipide, usw. Die Oberfläche der Liposomen kann weiterhin mit einem Polymer modifiziert sein, beispiels-weise mit Polyethylenglycol.

Es bedarf keiner besonderen Erwähnung, dass die erfindungsgemäße Steuervorrichtung noch weitere Messwerterfassungen von einem Patienten (beispielsweise akustisch evozierte Potentiale usw.) umfassen kann, um den angesteuerten und angestrebten Zustand genauer kontrollieren zu können. Da Edelgase nur über die Lunge durch die Ventilation eliminiert werden, ergibt sich bei der intravenösen Verabreichung der edelgashaltigen Präparation allerdings zum ersten Mal die Möglichkeit, über die Messung der endexpiratorischen Konzentration des Edelgases (insbesondere Xenon) die tatsächliche Konzentration eines intravenös verabreichbaren Pharmakons kontinuierlich zu bestimmen. Dadurch kann man über die Ansteuerung verschiedener reeller arterieller Konzentrationen sowohl die Narkosetiefe, als auch die Analge-sietiefe und gewünschtenfalls zusätzlich die Muskelrelaxation präzise ansteuern. Die Erfindung ermöglicht also eine wirkliche "Target-Controlled" Anästhesie. In diesem Fall sind keine mathematischen Modelle für die effektive Plasmakonzentration erforderlich.

Da die Elimination ausschließlich über die Lunge erfolgt, ist eine exakte Steuerung der Narkose sogar bei Patienten mit eingeschränkter Organfunktion, d.h. beispielsweise Leberund/oder Nierenfunktionsstörung - möglich.

Gegenstand der Erfindung ist weiterhin eine Vorrichtung zur Steuerung einer Narkose, wobei die intravenöse Zufuhr der edelgashaltigen Infusionslösung in Abhängigkeit von der Edelgaskonzentration in der von dem Patienten ausgeatmeten Luft eingestellt wird.

Die Messung der Edelgaskonzentration in der ausgeatmeten Luft kann besonders einfach, insbesondere bei Xenon, mit einem Gasdetektor erfolgen.

Diese Vorrichtung zeichnet sich dadurch aus, dass sie apparativ besonders einfach ist. Sie kann insbesondere bei der Katastro-phenmedizin zum Einsatz kommen, für die Einrichtungen mit geringem Platzbedarf besonders vorteilhaft sind.

Diese Vorrichtung kann auch Bestandteil einer Anlage sein, die zur Überwachung/Steuerung einer Narkose mit einem gasförmigen Anästhetikum zum Einsatz kommt.

Gegenstand der Erfindung ist weiterhin eine Vorrichtung zur Sedierung, insbesondere Analgosedierung, mit (a) einer Einrichtung, die eine Emulsion mit einem sedierend wirkenden Gehalt an Xenon bereitstellt, (b) einer Messdatenerfassung, die von einem Patienten Messdaten aufnimmt, welche einen Rückschluß auf den Zustand des Patienten erlaubt, und (c) einer Steuerung, die in Abhängigkeit von den erfassten Daten die Abgabe der Emulsion aus der Einrichtung an den Patienten steuert. Eine Vorrichtung zur (kontrollierten) Analgosedierung kann insbesondere im Rahmen der Intensivtherapie und nach Herzoperationen sinnvoll sein. Diese Vorrichtung umfasst einen Perfusor, gegebenenfalls eine expiratorische Xenonmessung und einen Pulsoximeter. Vorteilhaft ist, dass bei der Sedierung zugleich eine Analgesie erreicht werden kann.

Zusammengefaßt, die erfindungsgemäße Vorrichtung lässt sich vielseitig einsetzen, insbesondere bei der Intensivtherapie, der Endoskopie, der Zystoskopie, bei oberflächlichen Eingriffen und bei Herzoperationen.

Gegenstand der Erfindung ist darüber hinaus eine Vorrichtung zur gesteuerten Narkose, wobei die Konzentration des Xenons in einer Präparation, die einem Patienten intravenös zugeführt wird, in Abhängigkeit von der Xenon-Konzentration in der ausgeatmeten Luft eines narkotisierten Patienten geregelt wird. Eine solche Vorrichtung weist gegebenenfalls eine Mischvorrich-tung auf, in der die Präparation mit dem Xenon vermischt wird. Diese Mischvorrichtung kann temperaturgesteuert sein (Bereich von 1°C bis 35°C). (Die Präparation kann auch bereits vorher mit Xenon beladen worden sein.) Hierbei kommt es, wie bereits zuvor beschrieben, zu einer weitgehenden Auflösung des Xenons. Im einfachsten Fall kann die Mischvorrichtung aus einem Gefäß bestehen, durch das die Präparation hindurchgeleitet wird, und das z. T. von einer für Xenon durchlässigen semipermeablen Membran umgeben ist. Die Konzentration des Xenons in der Präparation wird dann im wesentlichen durch den Xenondruck auf die semipermeable Membran bestimmt. Zusätzlich sind hier auch zur Verbesserung der Auflösung und bzw. oder der Dispergierung des Xenongases weitere Hilfsmittel denkbar, beispielsweise aktive oder passive Rührelemente. Die Auflösung bzw. Dispergierung des Xenons lässt sich auch durch Ultraschallbestrahlung verbessern. Durch einfache Beobachtung des Patienten lässt sich nun während einer Vollnarkose leicht die Xenon-Konzentration in der ausgeatmeten Luft bestimmen, bei der eine ausreichende Narkose noch vorliegt. Soweit die adäquate Narkosetiefe unterschritten wird, kann man dem durch höhere Xenongaben mittels der Präparation begegnen. Die Xenonzufuhr über die Präparation lässt sich nun durch die Xenonbeladung und die Infusionsrate (mittels konventioneller Infusionspumpen z. B.) steuern. Auf diese Weise ist quasi eine Feinsteuerung der Narkose möglich, wie sie mit intravenösen Narkosemitteln bis zum heutigen Tage nicht erreicht werden konnte.

Gegenstand der Erfindung ist weiterhin eine Vorrichtung, die der zuvor beschriebenen Vorrichtung weitgehend entspricht, wobei aber keine Zumischung von Xenon vorgesehen ist. In einem solchen Fall ist die Präparation immer bereits mit Xenon beladen und die Narkose wird dadurch gesteuert, dass die Infusionsrate bzw. die Konzentration des Xenons in Abhängigkeit von der gemessenen expiratorischen Xenon-Konzentration eingestellt wird. Die Konzentration des Xenons in der Präparation kann beispielsweise dadurch verringert werden, dass eine weitere Präparation zugemischt wird, die kein Edelgas enthält. Auch hier erfüllen einfache Infusionspumpen (ggf. peristaltische Pumpen) den erfindungsgemäßen Zweck. Sowohl bei dieser Vorrichtung als auch bei der zuvor beschriebenen Vorrichtung mit einer Xenon-Einmischung in die Präparation kann eine Temperatursteuerung vorgesehen sein.

Die Fig.1 zeigt eine Spritze, wie sie im Prinzip erfindungsgemäß eingesetzt werden kann. Bei dieser sehr einfachen Ausführungsform der Erfindung kann die Spritze, die eine xenonhaltige Präparation enthält, als Einrichtung aufgefasst werden, die eine Präparation mit einem anästhesierend, analgesierend oder sedierend wirksamen Gehalt eines lipophilen Edelgases bereitstellt.

Die Fig.2 zeigt als erfindungsgemäße Vorrichtung einen befüllten Infusionsbeutel mit einem Regler.

Die Fig.3 zeigt eine erfindungsgemäße Vorrichtung, die einen gefüllten Infusionsbeutel mit einem Ableitungsschlauch und einen einfachen Regler zur Steuerung der Abgabe aufweist.

Die Fig.4 zeigt eine weitere erfindungsgemäße Vorrichtung.

Die Fig.5 zeigt schematisch eine erfindungsgemäße Vorrichtung zur Sedierung (sogenannte closed-loop Anordnung).

Die Fig.6 zeigt eine erfindungsgemäße Vorrichtung, die Teil einer Vorrichtung zur Durchführung einer Narkose mit einem Gas ist und die eine Xenonmessung in der ausgeatmeten Luft einschließt.

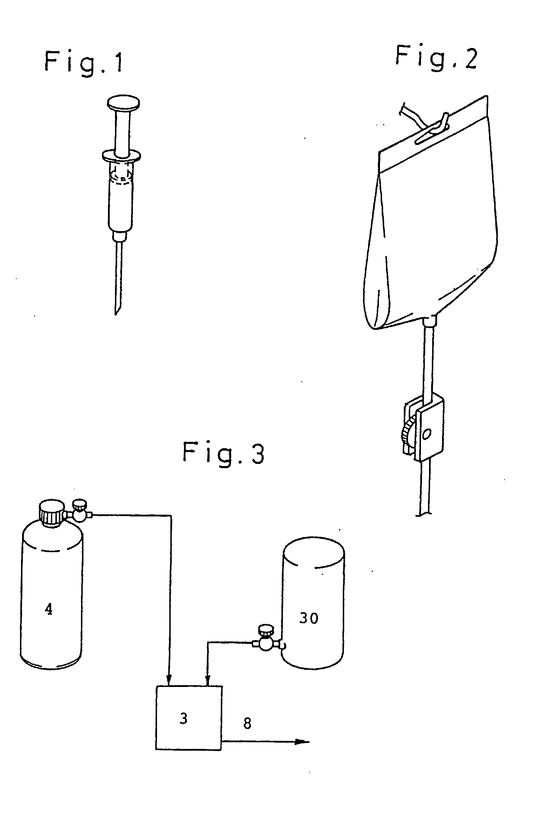

Die erfindungsgemäße Vorrichtung zur Durchführung einer gesteuerten Narkose wird anhand der schematischen Zeichnung (Fig.3) näher erläutert.

Diese Vorrichtung umfasst einen Vorratsbehälter 30 für eine flüssige Präparation, die Xenon in gelöster Form aufnehmen kann, und einen Gasbehälter 4 für das Edelgas sowie eine Mischungsvorrichtung 3, in der die Präparation mit dem Edelgas vermischt wird. Nicht abgebildet sind Steuerungsvorrichtungen (Infusionspumpen, Regler etc.), mit denen die intravenöse Zugabe an den Patienten gesteuert wird.

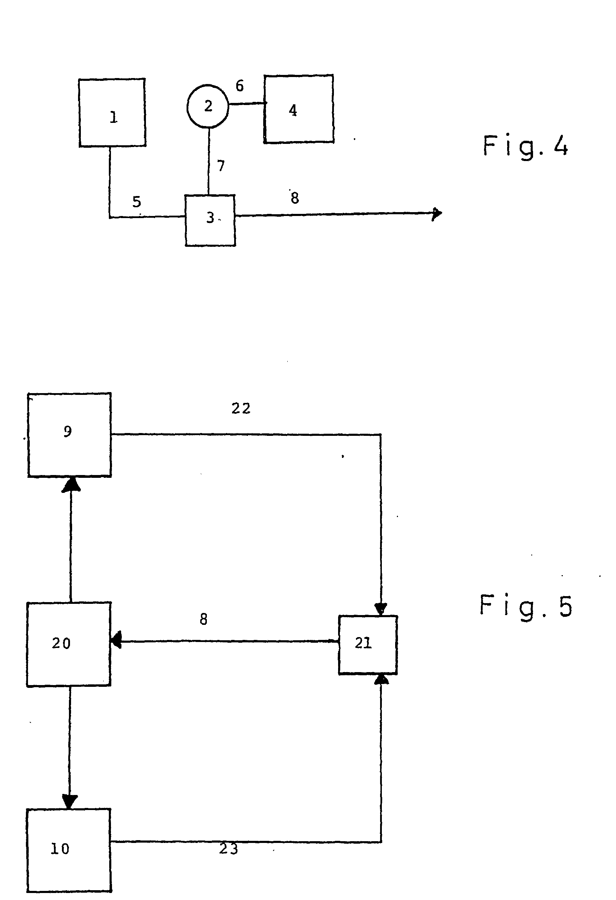

Eine erfindungsgemäße Vorrichtung wird weiterhin anhand der schematischen Zeichnung (Fig.4) näher erläutert. Diese Vorrichtung kann für eine gesteuerte Narkose eingesetzt werden, wobei die Xenon-Konzentration in der von einem narkotisierten Patienten ausgeatmeten Luft analytisch erfasst wird und in Abhängigkeit von diesem Analysenwert die Xenon-Konzentration in einer Präparation, die intravenös dem Patienten zugeführt wird, eingestellt wird. Die Vorrichtung umfasst folglich einen ggf. temperierbaren Vorratsbehälter 1 (Temperaturbereich 1°C bis 35°C) für die Präparation, der über eine Leitung 5 mit einer wiederum ggf. temperierbaren Mischvorrichtung 3 in Verbindung steht. In der Mischvorrichtung 3 wird das Xenon, das aus einer Xenon-Flasche 4 über die Leitung 6, die Dosiersteuereinheit 2 und die weitere Leitung 7 in die Mischvorrichtung 3 gelangt, mit der Präparation vermischt. Dabei löst sich das Xenon zum größten Teil in der Emulsion auf. Die xenonhaltige Präparation gelangt dann über die Leitung 8 und einen venösen Zugang in einen zu narkotisierenden Patienten. Der Transport der Präparation wird mittels an sich bekannter (nicht abgebildeter) Pumpen gewährleistet. Im medizinischen Bereich bedient man sich hier im einfachsten Fall sog. peristaltischer Pumpen. Solche Pumpen können bei der erfindungsgemäßen Vorrichtung beispielsweise in der Leitung 5 und zusätzlich Leitung 8 vorgesehen sein. Nicht abgebildet ist die endexpiratorische Gassensorik und Probenentnahme. Üblicherweise entnimmt man das ausgeatmete Gas am Tubusansatz oder in Mundnähe bei Maskenbeatmung oder Maskenoxygenierung beim In- und Exspirium des Patienten. Verfahren zur Bestimmung der Xenon-Konzentration in der ausgeatmeten Luft sind allgemein bekannt (Gasdetektoren und dgl.).

(Nicht abgebildet sind ferner diverse Ventile, die den Zufluß und Abfluß von Lösungen und Gasen regeln können).

Die Fig.5 zeigt schematisch eine erfindungsgemäße Vorrichtung zur Sedierung mit einer closed-loop Steuerung. Hier wird zum einen durch Messung des Edelgases in der ausgeatmeten Luft die effektive Xenon-Konzentration im Blut gemessen. Zum anderen werden weitere Messdaten von dem Patienten aufgenommen (beispielsweise akustisch evozierte Potentiale). Beide Meßergebnisse werden zur Steuerung der Perfusionspumpe eingesetzt.

Die von einem Patienten 20 aufgenommenen Daten hinsichtlich der Xenon-Konzentration in der ausgeatmeten Luft (Xenon-Sensor/Detektor 9) und der akustisch evozierten Potentiale (Aufzeichnungsvorrichtung 10) werden über die Datenleitungen 22 und 23 an die Perfusionspumpe 21 abgegeben. Die Messdaten werden verarbeitet (beispielsweise mittels eines Rechners) und in die erforderliche Infusionsrate umgesetzt, mit der die xenonhaltige Präparation über die Leitung 8 in den Patienten gelangt. Anders ausgedrückt, über die erfassten Messdaten wird die Perfusions-pumpe 21 angesteuert, die wiederum die Infusionsrate bestimmt. Die hier veranschaulichte Vorrichtung ist selbstverständlich nur ein Schema und eine tatsächliche Vorrichtung umfasst Anzeigen und Regler etc., wie sie üblicherweise vorgesehen werden, um beispielsweise auch einen manuellen Eingriff in die Steuerung zu erlauben. Nicht abgebildet sind die Zuleitung zu der Perfusionspumpe und ein Vorratsbehälter, über den eine xenonbeladene Präparation bereitgestellt wird.

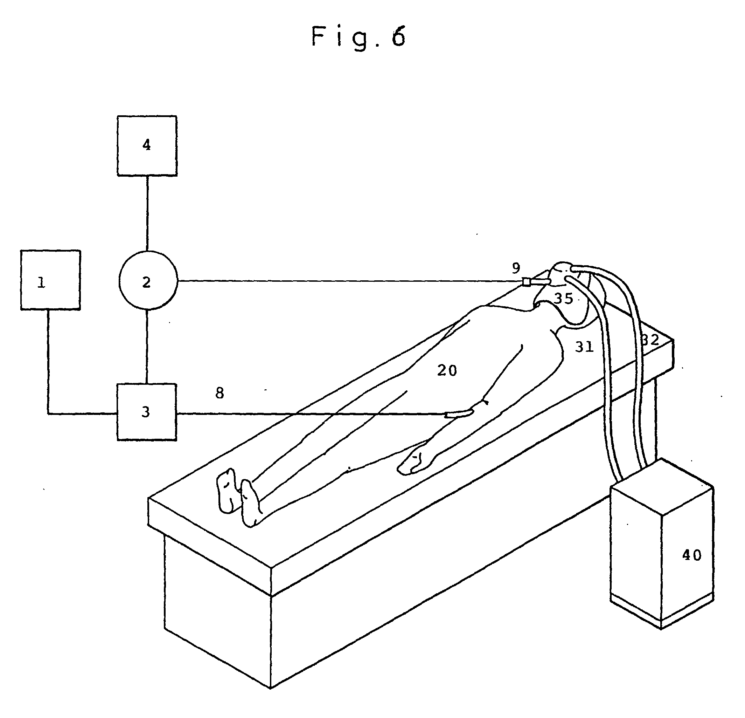

Die Fig.6 zeigt schematisch, wie eine erfindungsgemäße Vorrichtung in eine allgemeine Narkosesteuerung eingebunden werden kann. Diese Vorrichtung umfasst einen Vorratsbehälter 1 für die Präparation, die Mischvorrichtung 3, eine Xenonflasche 4 und eine Dosiersteuereinheit 2. Die Dosiersteuereinheit 2 ist mit einem Xenondetektor 9 verbunden, der die in der endexpira-torischen Luft gemessene Xenonkonzentration mißt und einen Meßwert an die Dosiersteuereinheit 2 abgibt. Über die Dosier-steuereinheit 2 wird dann die Zudosierung des Xenons in die flüssige Präparation gesteuert. Von der Mischeinrichtung 3 gelangt dann die xenonhaltige Präparation über den Infusionsschlauch 8 in den Patienten. Über die Xenonkonzentration in der ausgeatmeten Luft lässt sich selbstverständlich auch die Infusionsrate ansteuern.

Daneben ist noch eine Steuerungsvorrichtung 40 für die Zuleitung eines gasförmigen oder eines Inhalationsanästhetikums vorhanden. Diese Vorrichtung umfasst Zuleitungs- und Ableitungsschläuche 31 und 32 für die Zu- und Ableitung des Narkosegases über die Inhalationsmaske 35.

### Experimenteller Teil

### Fettemulsionen

In den folgenden Beispielen wurden als Fettemulsionen die käuflich erhältlichen Intralipidpräparate (erhältlich von Pharmacia & Upjohn GmbH, Erlangen) verwendet. Diese Emulsionen bestehen im wesentlichen aus Sojabohnenöl, 3-sn-Phosphatidylcholin (aus Hühnereigelb) und Glycerol. Eine Intralipid®10 Fettemulsion hat beispielsweise die folgende Zusammensetzung:

| | |
|---|---|
| Sojabohnenöl (3-sn-Phosphatidyl)cholin | 100 g |
| aus Hühnereigelb | 6 g |
| Glycerol | 22,0 g |
| Wasser für Injektionszwecke ad | 1000 ml |

Mit Natriumhydroxid auf pH 8,0 eingestellt.
Energiewert/l: 4600 kJ (1100 kcal)
Osmolarität: 260 mOsm/l
Eine/Intralipid®20 Fettemulsion hat beispielsweise die folgende Zusammensetzung:

| | |
|---|---|
| Sojabohnenöl (3-sn-Phosphatidyl)cholin | 200 g |
| aus Hühnereigelb | 12 g |
| Glycerol | 22,0 g |
| Wasser für Injektionszwecke ad | 1000 ml |

Mit Natriumhydroxid auf pH 8,0 eingestellt.
Energiewert/l: 8400 kJ (2000 kcal)
Osmolarität: 270 mOsm/l

### Beladung von Perfluorcarbonemulsionen mit Xenon

Es wurde eine Reihe von Perfluorcarbonemulsionen hergestellt bzw. käuflich erworben und mit Xenon beladen. Die Wirksamkeit der Präparationen wurde am Tiermodell verifiziert (Kaninchen). Alle Emulsionen verhielten sich so wie die zuvor beschrieben Intralipidpräparate, d.h. das Versuchstier wurde durch eine Injektion in das Ohr kurzzeitig narkotisiert (etwa 1 ml).

Die Emulsionen wurden jeweils in einem Becherglas vorgelegt und durch Durchleiten des Xenongases beladen.

Folgende Perfluorcarbonverbindungen wurden eingesetzt: Perfluorhexyloctan (1), Perfluordecalin (2), Perflubron (C₈F₁₇) (3).

Darüber hinaus wurden zur Herstellung der Emulsionen Emulgatoren eingesetzt, beispielsweise Eidotterlecithin (Lipoid E100 von der Lipoid GmbH, Ludwigshafen), Pluronic PE6800 und Pluronic F68.

Es wurde bei allen Emulsionen festgestellt, dass bereits eine 40 %ige Perfluorcarbonemulsion (Gewicht/Volumen; d.h. Gewicht Perfluorcarbonverbindung zu Volumen Emulsion) 1 bis 4 ml Xenon pro ml Emulsion aufnehmen konnte.

### Tierexperimentelle Studien

Um zu zeigen, dass eine Steuerung der Anästhesie, hier Aufrechterhaltung der Anästhesie, erfindungsgemäß möglich ist, wurde eine Untersuchung mit 24 Schweinen, Alter 14 bis 16 Wochen, mit einem Gewicht von 36,4 -43,6 kg durchgeführt. Insgesamt wurden zufallsbestimmt 3 Gruppen gebildet, die anästhesiert wurden. In allen Gruppen wurde die Anästhesie mit einer Bolusinjektion Pentobarbiton (8 mg/kg Körpergewicht) und Buprenorphin (0,01 mg/kg Körpergewicht) intravenös eingeleitet. Bei einer Gruppe (Vergleichsgruppe) wurde die Anästhesie durch intravenöse Applikation von 2,6-Diisopropylphenol (10 mg/l ml Emulsion) aufrechterhalten. Zwei Gruppen (erfindungsgemäß) Schweine zu je vier Individuen erhielten zur Aufrechterhaltung der Anästhesie eine Infusion intravenös von jeweils 1 ml/kg/h einer 10 gew.-%igen erfindungsgemäßen Fettemulsion, die mit Xenon zuvor gesättigt wurde (etwa 0,3 ml Xenon pro ml Emulsion). In der Gruppe 2 wurde zusätzlich 7,5 mg/kg Körper- gewicht/h 2,6-Diisopropylphenol mit der Fettemulsion appliziert.

Die Schweine wurden einem chirurgischen Eingriff (Standardeingriff: Auftrennung der linken femoralen Arterie) unterzogen (identisch in jeder Gruppe und bei jedem Versuchstier) und es wurde der Adrenalinspiegel, die Herzfrequenz, der Arterienblutdruck und der Sauerstoffverbrauch aufgezeichnet. Weiterhin wurde festgehalten, welche zusätzlichen Pentobarbitongaben erforderlich waren, um die Analgesie und Narkosetiefe in jeder Gruppe auf das erforderliche Level zu bringen.

**Tabelle**

| Gruppe | Adrenalin pg/ml | Herzfrequenz [min^{-1]} | Arterienblutdruck [mm Hg] | VO₂ [ml/min] | Pentobarbitonerfordernis mg/kg/min |
|---|---|---|---|---|---|
| Vergleichsgruppe | 60 | 115 | 110 | 410 | 0,25 |
| | 134 | 120 | 105 | 391 | 0,36 |
| | 112 | 105 | 115 | 427 | 0,31 |
| | 85 | 98 | 101 | 386 | 0,42 |
| | | | | | |
| Gruppe 1 | 38 | 112 | 112 | 341 | 0,09 |
| | 21 | 106 | 100 | 367 | 0,04 |
| | 16 | 95 | 104 | 348 | 0,11 |
| | 30 | 112 | 118 | 334 | 0,15 |
| | | | | | |
| Gruppe 2 | 10 | 88 | 100 | 325 | - |
| | 23 | 100 | 85 | 346 | - |
| | 14 | 94 | 93 | 331 | - |
| | 8 | 104 | 87 | 354 | - |

Die in der Tabelle angegebenen Werte zeigen, dass die xenonhaltige Präparation allen z.Zt. erhältlichen intravenösen Narkosemitteln insbesondere wegen der zusätzlichen analgetischen Potenz überlegen ist. So zeigen die Schweine in der Gruppe 1 (10 gew.%ige Fettemulsion, gesättigt mit Xenon) im Vergleich (siehe Vergleichsgruppe) deutlich weniger Streß (Adrenalinspiegel), geringeren Sauerstoffbedarf (VO₂) und ein geringeres Pentobarbitonerfordernis (also eine bessere Narkose). Der Unterschied zu intravenös zu applizierenden Narkosemitteln nach dem Stand der Technik zeigt sich noch deutlicher, wenn man die Ergebnisse in der Gruppe 2 (10 %ige Fettemulsion mit 2,6-Diisopropylphenol und angereichert mit Xenon) der Vergleichsgruppe gegenüberstellt. Hier zeigt sich nicht nur ein deutlich verminderter Streß (Adrenalinspiegel). Bei deutlich beruhigter Herzfrequenz und geringerem Arterienblutdruck sowie geringerem Sauerstoffbedarf konnte auf zusätzliche Pentobarbitongaben verzichtet werden.

Diese Studie zeigt, dass über den gesamten Verlauf der Narkose das angestrebte Ziel, hier Aufrechterhaltung der'Anästhesie, erreicht werden kann.

Bei einer weiteren Gruppe (4 Schweine ä 31,4 bis 39,8 kg Körpergewicht) wurde der Einsatz von Perfluorcarbonpräparationen untersucht. Bei dieser Versuchsgruppe wurde eine 40 %ige Perfluorcarbonemulsion mit einem Xenongehalt von 2,1 ml Xenon pro ml Emulsion eingesetzt. Zur Einleitung und Intubation erhielten die Schweine intravenös 20 ml der Emulsion über 20 sec (das entspricht 1,34 ml Xenon/kg Körpergewicht). Nach der Intubation und Beatmung wurde Xenon intravenös kontinuierlich über 30 min infundiert. Dabei erhielten die Versuchstiere insgesamt 75 ml Emulsion (das entspricht 10 ml Xenon kg⁻¹h⁻¹).

| Adrenalin [pg/ml] | Herzfrequenz [min⁻¹] | Arterienblutdruck [mm Hg] | VO₂ [ml/min] |
|---|---|---|---|
| 8 | 90 | 101 | 301 |
| 6 | 87 | 96 | 320 |
| 10 | 94 | 98 | 308 |
| 5 | 100 | 106 | 316 |

In der Tabelle oben sind die Messergebnisse für den Adrenalinspiegel, die Herzfrequenz, der Arterienblutdruck und den Sauerstoffverbrauch angegeben. Die Ergebnisse zeigen, dass bei höherer Xenonbeladung und größeren Infusionsraten (über 5 ml/kg/h) eine vollständige Narkose allein mit dem erfindungs-gemäßen Mittel durchgeführt werden kann. Hierbei wird insgesamt sogar noch ein geringerer Sauerstoffbedarf (VO₂) und eine streßfreiere Narkose (Adrenalinspiegel und Herzfrequenz) fest-gestellt.

## Patentansprüche

1. Vorrichtung zur gesteuerten Anästhesie, Analgesie und/oder Sedierung, **dadurch gekennzeichnet, daß** die Vorrichtung einen Behälter mit einer Emulsion, die Xenon in einer anästhesierend, analgesierend oder sedierend wirksamen Menge enthält, und Mittel zur gesteuerten Abgabe der Emulsion an einen Patienten aufweist.

2. Vorrichtung zur gesteuerten Anästhesie, Analgesie und/oder Sedierung, **dadurch gekennzeichnet, daß** die Vorrichtung einen Behälter mit einer flüssigen Präparation, die Xenon in einer anästhesierend, analgesierend oder sedierend wirksamen Menge enthält, und Mittel zur gesteuerten Abgabe der Praparation an einen Patienten, die einen Perfusor zur Steuerung der Infusionsrate der flüssigen Präparation umfassen, aufweist.

3. Vorrichtung zur gesteuerten Anästhesie, Analgesie und/oder Sedierung, **dadurch gekennzeichnet, daß** die Vorrichtung einen Behälter mit einer flüssigen Präparation, die Xenon in einer anästhesierend, analgesierend oder sedierend wirksamen Menge enthält, und Mittel zur gesteuerten Abgabe der Praparation an einen Patienten aufweist, wobei der Behälter ein Infusionsbeutel ist, verbunden mit einem Schlauch, der einen Regler zur Steuerung der Infusionsrate der flüssigen Präparation umfaßt.

4. Vorrichtung nach einem der Ansprüche 1, 2 oder 3 zur Durchführung einer gesteuerten Narkose, wobei die intravenöse Zufuhr einer Infusionslösung, die das Xenon enthält, in Abhängigkeit von der Xenonkonzentration in der von einem Patienten ausgeatmeten Luft eingestellt wird, mit einem Behälter (1) für die Infusionslösung und einer Dosiersteuerung (2), die die Infusionsrate steuert.

5. Vorrichtung nach Anspruch 4, wobei zusätzlich von einem Patienten Meßdaten aufgenommen werden, die einen Rückschluß auf die Narkosetiefe erlauben.

6. Vorrichtung nach Anspruch 1, wobei ein Vorratsbehalter (30) für eine flüssige Präparation, die Xenon in gelöster Form aufnehmen kann, einen Gasbehälter (4) für das Edelgas sowie eine Mischvorrichtung (3), in der die flüssige Präparation mit dem Xenon vermischt wird, vorgesehen sind.

7. Vorrichtung nach Anspruch 1 zur Sedierung mit
(a) einer Einrichtung, die eine flüssige Präparation mit einem sedierend wirksamen Gehalt an Xenon bereitstellt,
(b) einer Meßdatenerfassung, die von einem Patienten Meßdaten aufnimmt, welche einen Rückschluß auf den Zustand des Patienten erlauben, und
(c) einer Steuerung, die in Abhängigkeit von erfaßten Daten die Abgabe der Emulsion aus der Einrichtung an den Patienten steuert.

8. Vorrichtung nach einem der vorhergehenden Ansprüche, **dadurch gekennzeichnet, daß** neben dem Xenon ein weiteres pharmakologisch wirksames Mittel enthalten ist.

## Claims

1. Device for controlled anaesthesia, analgesia and/or sedation, **characterized in that** the device comprises a container holding an emulsion which contains xenon in an amount effective as an anaesthetic, analgesic or sedative, and means for the controlled administration of the emulsion to a patient.

2. Device for controlled anaesthesia, analgesia and/or sedation, **characterized in that** the device comprises a container holding a liquid preparation which contains xenon in an amount effective as an anaesthetic, analgesic or sedative, and means for the controlled administration of the preparation to a patient which comprise a perfuser for controlling the infusion rate of the liquid preparation.

3. Device for controlled anaesthesia, analgesia and/or sedation, **characterized in that** the device comprises a container holding a liquid preparation which contains xenon in an amount effective as an anaesthetic, analgesic or sedative, and means for the controlled administration of the preparation to a patient wherein the container is an infusion bag and has a tube and a regulator for controlling the infusion rate of the liquid preparation.

4. Device according to any of claims 1, 2 or 3 for carrying out controlled anaesthesia, wherein the intravenous supply of an infusion solution containing the xenon is adjusted as a function of the xenon concentration in the air exhaled by a patient, comprising a container (1) for the infusion solution and a metering means (2) for controlling the infusion rate.

5. Device according to claim 4 wherein additionally experimental data of a patient are recorded, allowing a conclusion to be drawn about the depth of anaesthesia.

6. Device according to claim 1 wherein provision is made for a storage container (30) for a liquid preparation which can take up xenon in dissolved form, a gas container (4) for the inert gas, and a mixer (3), in which the liquid preparation is mixed with the xenon.

7. Device according to claim 1 for inducing sedation, comprising
(a) a facility which provides a liquid preparation containing xenon in an amount active as a sedative,
(b) a means of measuring data, which records a patient's data, said data allowing a conclusion to be drawn about the patient's condition, and
(c) a control means which controls the administration of the emulsion from the facility to the patient as a function of the measured data.

8. Device according to any of the preceding claims **characterized by** comprising in addition to xenon a further pharmaceutically active agent.

## Revendications

1. Appareil pour l'anesthésie, l'analgésie et/ou la sédation contrôlée, **caractérisé en ce que** l'appareil présente un conteneur empli d'une émulsion renfermant du xénon dans une quantité anesthésique, analgésique ou sédative, et des moyens permettant l'administration contrôlée de ladite l'émulsion au patient.

2. Appareil pour l'anesthésie, l'analgésie et/ou la sédation contrôlée, **caractérisé en ce que** l'appareil présente un conteneur empli d'une préparation liquide renfermant du xénon dans une quantité anesthésique, analgésique ou sédative, et des moyens permettant l'administration contrôlée de ladite préparation au patient et comprenant un perfuseur pour le contrôle de la vitesse de perfusion de la préparation liquide.

3. Appareil pour l'anesthésie, l'analgésie et/ou la sédation contrôlée, **caractérisé en ce que** l'appareil présente un conteneur empli d'une préparation liquide renfermant du xénon dans une quantité anesthésique, analgésique ou sédative, et des moyens permettant l'administration contrôlée de ladite préparation au patient, le conteneur étant un sac de perfusion relié par un flexible incorporant un dispositif régulateur pour le contrôle de la vitesse de perfusion de la préparation liquide.

4. Appareil selon l'une des revendications 1, 2 ou 3 pour la réalisation d'une anesthésie contrôlée, l'apport par voie intraveineuse de la solution de perfusion renfermant le xénon
étant régulé en fonction de la concentration de xénon dans l'air expiré par le patient, présentant un conteneur (1) empli d'une solution de perfusion et un dispositif doseur (2) pour le contrôle de la vitesse de perfusion.

5. Appareil selon la revendication 4, les valeurs mesurées sur le patient qui renseignent sur la profondeur de l'anesthésie étant en outre enregistrées.

6. Appareil selon la revendication 1, un réservoir (30) destiné à contenir une préparation liquide susceptible de renfermer du xénon sous forme dissoute, un récipient à gaz (4) destiné à contenir le gaz rare et un mélangeur (3) dans lequel la préparation liquide est mélangée au xénon, étant prévus.

7. Appareil selon la revendication 1 pour la sédation au moyen
(a) d'un dispositif contenant une préparation liquide laquelle renferme une teneur en xénon apte à avoir un effet sédatif,
(b) d'une unité de saisie de données qui enregistre les valeurs mesurées sur le patient, lesquelles renseignent sur l'état du patient, et
(c) d'une commande qui contrôle l'administration de l'émulsion au patient depuis ledit dispositif en fonction des données saisies.

8. Appareil selon l'une des revendications précédentes, **caractérisé en ce qu'**il est, en plus du xénon, utilisé un autre agent pharmacologiquement actif.
